# EUROPÄISCHE PATENTSCHRIFT

(11) **EP 0 295 582 B1**
(45) Veröffentlichungstag und Bekanntmachung des Hinweises auf die Patenterteilung: **18.08.1993**
(21) Anmeldenummer: 88109261.3
(22) Anmeldetag: 10.06.1988
(51) Int. Cl.: C07D 319/12, C07C 41/22

(54) **Verfahren zur Herstellung von Chlor, Brom oder Iod sowie Ethersauerstoff enthaltenden, weitgehend perfluorierten Alkylverbindungen**
Process for the preparation of largely perfluorinated alkyl compounds containing chlorine, bromine or iodine
Procédé de préparation de composés alkyls largement perfluorés contenant du chlore, du brome, ou de l'iode

(30) Priorität: 19.06.1987 DE 3720323
(43) Veröffentlichungstag der Anmeldung: 21.12.1988
(73) Patentinhaber: HOECHST AKTIENGESELLSCHAFT, 65926 Frankfurt am Main (DE)
(72) Erfinder: von Werner, Konrad, Dr., D-8261 Halsbach (DE)

(56) Entgegenhaltungen:
- EP-A- 0 062 325
- FR-A- 1 323 803
- US-A- 4 085 137
- THE JOURNAL OF ORGANIC CHEMISTRY, Band 47, Nr. 11, Seiten 1985-2234, American Chemical Society; Z. CHENGXUE et al.: "Thermal decomposition of some perflouro- and polyfluorodiacyl peroxides"
- CHEMICAL ABSTRACTS, Band 106, Nr. 17, 27. April 1987, Seite 641, Zusammenfassung Nr. 137912a, Columbus, Ohio, US; W. HUANG et al.: "Sulfinatodehalogenation: IX. Synthesis and reaction of secondary perfluoroalkyl iodides"

## Beschreibung

Die Erfindung betrifft ein Verfahren zur Herstellung von Chlor, Brom oder Iod sowie Ethersauerstoff enthaltenden, weitgehend perfluorierten Alkylverbindungen nach Anspruch 1.

Es ist bekannt, aus Silbersalzen aliphatischer Carbonsäuren durch Umsetzung mit Halogenen wie Chlor, Brom oder Iod die entsprechenden Alkyl-Halogenide herzustellen. Anstelle der Silbersalze können auch Quecksilber- oder Kaliumsalze verwendet werden, doch hat deren Anwendung Nachteile. Die Kaliumsalze reagieren verhältnismäßig langsam, es ist daher längeres Erhitzen notwendig, wobei unerwünschte Nebenreaktionen wie Esterbildung und Substitution auftreten, so daß eine ungünstige Ausbeute beobachtet wird (Hunsdiecker & Hunsdiecker Ber. 75, Seiten 291 bis 293).

Diese Reaktion ist auch auf fluorierte, aliphatische Carbonsäuren angewendet worden, zur Herstellung von fluorierten Brom- oder Iod-Alkanen, wobei in der Regel ohne Lösungsmittel gearbeitet wurde. Zum Beispiel wurden bei Einsatz der Kalium- oder Natriumsalze der Trifluoressigsäure Ausbeuten von 40 bis 61 % Trifluoriodmethan erzielt, während die Kalium- und Natriumsalze längerkettiger Säuren wie Pentafluorpropionsäure oder Heptafluorbuttersäure nur zu Ausbeuten von 5 bis 32 % an den entsprechenden, fluorierten Mono-Brom beziehungsweise Mono-Iod-Produkten führten. (Johnson & Ingham, Chem. Rev. 56, Seite 219 ff., insbesondere Seite 238).

Wei-Yuan Huang und Wei Wang, Huaxue Xuebao, 44 (1986), Seiten 940 bis 945 = Acta Chimica Sinica Nr. 3 (1986), Seiten 266 und 270 stellen die Verbindung CF₃(CF₂)₂OCFICF₃ durch Umsetzung des Silbersalzes der α-Trifluormethyl-2-oxaoctafluorhexansäure mit Iod im Molverhältnis 1 : 1,72 bei 160 °C her. Das Verfahren ist durch die Verwendung eines Silbersalzes aufwendig, die erreichte Ausbeute im Vergleich zu den Möglichkeiten des weiter unten beschriebenen neuen Verfahrens deutlich schlechter (siehe Beispiele 1 und 2).

Zur Herstellung der Di-Iodverbindungen perfluorierter Ether aus den entsprechenden perfluorierten Etherdicarbonsäuren, ist aus US 4,085,137 lediglich bekannt, daß letztere mit Silberoxid und Iod umgesetzt werden sollen, jedoch ohne Angaben über die Umsetzungsbedingungen und die erhaltenen Produkte. Es ist ferner aus EP 62 325-A2 bekannt, Verbindungen der Formel 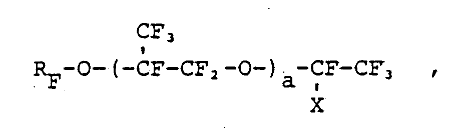
worin X unter anderem Brom oder Iod sein kann, R_{F} ein mono- oder difunktioneller Initiatorrest ist und a eine Zahl größer als 200 bedeutet, durch die Hunsdiecker-Reaktion mit dem entsprechenden Halogen und dem Silbersalz der mit einer Carboxylgruppe endenden Polymeren-Verbindung zu erhalten. Auch hier fehlen genauere Angaben über Reaktionsbedingungen und die erhaltenen Produkte.

Es wurde nun ein Verfahren zur Herstellung einer Chlor, Brom oder Iod sowie Ethersauerstoff enthaltenden, weitgehend perfluorierten Alkylverbindung gefunden, das ohne Verwendung des kostenaufwendigen Silbersalzes und ohne die damit verbundene Notwendigkeit der Rückgewinnung des Silbers unter leicht einzustellenden Reaktionsbedingungen zu einem Produkt mit guter Ausbeute führt. Das neue Verfahren zur Herstellung einer Verbindung der Formel 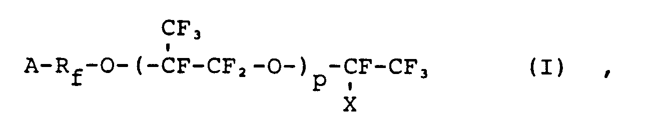
in der bedeuten A = H; F; Cl; Br; I oder 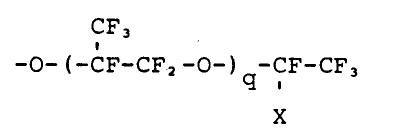
- R_{f}: einen Perfluoralkylenrest mit 2 bis 10 Kohlenstoffatomen oder A und R_{f} zusammen einen Perfluor-3,6-dimethyl-1,4-dioxanyl-(2)-Rest
- p =: Null oder eine Zahl von 1 bis 10
- q =: Null oder eine Zahl von 1 bis 10
- X =: Cl; Br oder I
durch Umsetzung eines Salzes einer Verbindung der Formel 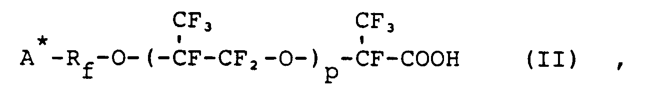
in der bedeuten
- A*=: H; F; Cl; Br; I oder 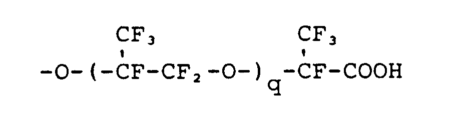
- R_{f}: denselben Perfluoralkylenrest wie oben oder A* und R_{f} zusammen einen Perfluor-3,6-dimethyl-1,4-dioxanyl-(2)-Rest,
- p und q: haben die gleiche Bedeutung wie oben,
mit einem Halogen bei Zimmertemperatur oder erhöhter Temperatur in Abwesenheit von Wasser, ist dadurch gekennzeichnet, daß als Salz ein Alkali-, Erdalkali- oder quaternäres Ammonium-Salz der Verbindung der Formel (II) und als Halogen Chlor, Brom, Iod oder mindestens eine Verbindung, die unter den gewählten Reaktionsbedingungen Chlor, Brom oder Iod entwickelt, eingesetzt werden.

Die Verbindungen der Formel (II) sind bekannt. Sie können beispielsweise hergestellt werden nach Verfahren, wie sie beschrieben sind in US 3,205,807; US 3,205,808; DE-OS 24 34 992 sowie von H. Millauer; W. Schwertfeger und G. Siegemund,Angew. Chem. 97, Seite 164 ff, insbesondere 168 und 169. Sofern bei den Verfahren die Säurefluoride erhalten werden, werden diese nach bekannten Verfahren verseift und zur Erzeugung der Alkali-, Erdalkali- oder quaternären Ammoniumsalze mit den entsprechenden Hydroxiden neutralisiert wie beispielsweise in DE-OS 25 17 357 beschrieben, wobei die Salze jedoch nachfolgend nicht auf über 150 °C erhitzt werden. Die Salze werden zweckmäßig,beispielsweise durch Vakuumtrocknung,weitgehend von Wasser befreit. Sofern die nachfolgende Umsetzung in einem aprotischen Lösungsmittel durchgeführt wird, können die Salze zweckmäßig in diesem Lösungsmittel gelöst und über üblichen Trockenmitteln, beispielsweise Kieselgel oder einem sogenannten Molekularsieb, getrocknet werden. Eine weitere Möglichkeit,wasserfreie Salze zu erzeugen, ist die Umsetzung der Säurefluoride mit dem entsprechenden wasserfreien Alkali , Erdalkali- oder quaternären Ammoniumcarbonat.

Bevorzugt werden für das erfindungsgemäße Verfahren die Lithium-, Natrium-, Calcium-, Magnesium- oder quaternären Ammonium-Salze verwendet. Als letztere sind beispielsweise geeignet Alkyl- oder Aralkylammonium-Salze, die im Ammoniumkation 4 bis 24 Kohlenstoffatome enthalten. Die Kohlenstoffketten der Alkylsubstituenten am Stickstoff des Ammoniumkations können durch Ethersauerstoffatome unterbrochen sein. Besonders gute Ergebnisse werden mit dem Kaliumsalz der Verbindung der Formel (II) erhalten.

Wegen der leichten Zugänglichkeit und günstigen Reaktionsergebnisse wird bevorzugt ein Salz der Verbindung der Formel (II) eingesetzt, das folgender Formel entspricht: 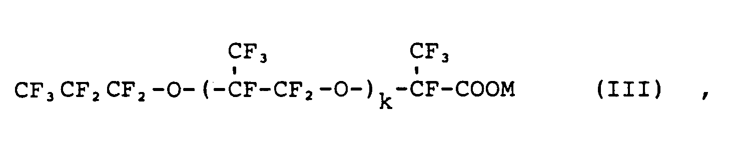
in der bedeuten:
- k =: Null oder eine Zahl von 1 bis 6 und
- M =: Li; Na; K; 1/2 Ca; 1/2 Mg oder quaternäres Ammonium.

Neben dem zweckmäßigerweise vorher getrockneten elementaren Chlor, Brom oder Iod kann auch mindestens eine Verbindung eingesetzt werden, die unter den gewählten Reaktionsbedingungen Chlor, Brom oder Iod entwickelt, beispielsweise Chlor-,Brom- oder Iod-Succinimid, es können auch Verbindungen der genannten Halogene untereinander wie Iodchlorid oder Bromchlorid Verwendung finden. Vorzugsweise werden je mol des Salzes der Verbindung der Formel (II) ein bis 1,2 mol Cl₂; Br₂ oder I₂ beziehungsweise die äquivalente Menge einer Verbindung,die eines dieser Halogene entwickelt, eingesetzt. Es kann auch außerhalb dieses Vorzugsbereiches gearbeitet werden, jedoch werden hier Ausbeuteverschlechterung und/oder zunehmende Nebenreaktionen festgestellt.

Das erfindungsgemäße Verfahren kann sowohl ohne wie auch mit Verwendung eines Lösungsmittels durchgeführt werden. In einer bevorzugten Ausführungsform wird mindestens ein polares aprotisches Lösungsmittel, das unter den Reaktionsbedingungen flüssig ist, eingesetzt. Geeignete Lösungsmittel sind beispielsweise offenkettige oder cyclische Säureamide wie Dimethylformamid; N,N-Dimethylacetamid oder N-Methylpyrrolidon, ferner ein Sulfon, beispielsweise Tetramethylensulfon oder auch Ether,wie oligomere Ethylenglykoldialkylether, wobei mit Säureamiden und Sulfonen besonders gute Ergebnisse erzielt werden. Zur Vermeidung von unerwünschten Nebenreaktionen soll das verwendete Lösungsmittel möglichst wasserfrei sein. Unter den vielen hierfür geeigneten Methoden sei lediglich beispielhaft die Destillation von N,N-Diemethylacetamid über Calciumhydrid und anschließendes Aufbewahren über einem Molekularsieb erwähnt, die es ermöglicht, das Lösungsmittel auf bis zu 0,01 Gew.-% Restfeuchte zu trocknen.

In einer weiteren bevorzugten Ausführungsform wird als Lösungsmittel für das erfindungsgemäße Verfahren mindestens eine perfluorierte Verbindung benützt, die unter den Reaktionsbedingungen flüssig ist und an der Reaktion nicht teilnimmt. Zweckmäßig wird eine solche Verbindung gewählt, deren Siedepunkt sich deutlich von dem herzustellenden Umsetzungsprodukt unterscheidet und das mit letzterem möglichst keine konstant siedenden Gemische bildet. Geeignete Lösungsmittel sind beispielsweise:Fluorkohlenstoff-Verbindungen wie 1,1,2-Trichlor-trifluorethan oder 1-Chlorperfluoroctan oder perfluorierte Ether, insbesondere verzweige Polyether, beispielsweise Perfluor-5,8-dimehtyl-3,6,9-trioxaundecan. Vorteilhaft werden je mol des Salzes der Verbindung der Formel (II) 100 bis 2 000 g des Lösungsmittels beziehungsweise des Lösungsmittelgemisches verwendet.

Die Reaktionstemperatur hängt stark vom verwendeten Salz der Verbindung der Formel (II),dem eingesetzten Halogen und dem gegebenenfalls verwendeten Lösungsmittel ab. Vorteilhaft werden Temperaturen von 20 bis 150 °C angewendet, wobei im allgemeinen bei Verwendung von Lösungsmitteln die erforderlichen Temperaturen niedriger liegen, vorteilhaft bei 30 bis 80 °C, als ohne Verwendung dieser Lösungsmittel. Ebenso wird bei Einsatz von Chlor im allgemeinen eine niedrigere Temperatur ausreichen als bei Verwendung von Brom oder Iod. Ein besonders geeigneter Temperaturbereich kann vom Fachmann leicht anhand der Kohlendioxidentwicklung aus dem Reaktionsgemisch festgestellt werden.

Die erforderliche Reaktionsdauer hängt von den eingesetzten Substanzen und der gewählten Reaktionstemperatur ab. Im allgemeinen ist die Reaktion in 0,5 bis 10 Stunden, vorzugsweise 1 bis 5 Stunden beendet. Das Ende der Reaktion kann leicht daran festgestellt werden, daß die Reaktionsmischung kein Kohlendioxid mehr entwickelt.

Um einen möglichst vollständigen Ausschluß von Feuchtigkeit zu erreichen, ist es zweckmäßig, mit einem trockenen Schutzgas, beispielsweise Stickstoff oder Argon, zu arbeiten, jedoch ist häufig auch getrocknete Luft ausreichend.

Das erfindungsgemäße Verfahren wird bei Normaldruck oder erhöhtem Druck im Bereich von 0,1 bis 1 MPa durchgeführt. Insbesondere bei Einsatz von Chlor oder Brom kann ein Überdruck vorteilhaft sein. Während der Umsetzung wird die Reaktionsmischung zweckmäßig bewegt, beispielsweise durch Rühren oder Schütteln. Als Materialien für die Apparateoberflächen, die mit der Reaktionsmischung in Kontakt kommen, sind Glas oder Emaille bevorzugt, auch Edelstähle sind geeignet. Die Zugabe der Halogene erfolgt entsprechend ihrem Aggregatzustand durch Aufdrücken, Zutropfen oder Eintrag als zerkleinerte feste Substanz. Insbesondere bei der Anwendung von Brom, Iod oder anderen das entsprechende Halogen entwickelnden festen oder flüssigen Verbindungen, können auch Lösungen beziehungsweise Mischungen mit einem oder mehreren der obengenannten Lösungsmittel eingesetzt werden.

Aus dem Umsetzungsgemisch wird nach Beendigung der Reaktion das erfindungsgemäß erzeugte Produkt, beispielsweise durch fraktionierte Destillation, abgetrennt und gereinigt. Sofern wassermischbare Lösungsmittel verwendet werden, kann die Abtrennung auch vorteilhaft durch Zugabe von Wasser zur Reaktionsmischung erfolgen. Das so abgetrennte Produkt wird zweckmäßig nach Trocknung einer weiteren Reinigung durch Destillation unterworfen. Bei höher-molekularen Produkten können entsprechend andere Reinigungsverfahren, wie Auflösen und wieder Ausfällen beziehungsweise Umkristallisieren, angewendet werden.

Wie eingangs erwähnt,ermöglicht es das erfindungsgemäße Verfahren, Verbindungen der Formel (I), ohne Einsatz der kostspieligen Silbersalze und einer dadurch notwendigen aufwendigen Wiedergewinnung des Silbers, herzustellen. Das neue Verfahren ermöglicht es, mit leicht einzustellenden Reaktionsbedingungen Produkte in guten Ausbeuten zu erhalten.

### Nachfolgende Beispiele sollen die Erfindung näher erläutern:

Die hergestellten Verbindungen der Formeln I; IV; V und VI werden durch Kernresonanzspektren identifiziert. Diese Spektren werden in CDCl₃-Lösung gemessen. ¹⁹F-NMR: 75,393 MHz, mit Trifluoressigsäure als externem Standard. ¹³C-NMR: 50,323 MHz, mit Tetramethylsilan als internem Standard. Die UV-Spektren der Iod-Verbindungen werden in n-Hexan-Lösung registriert. Fraktionierte Destillationen werden mit einer 30 cm-Spaltrohrkolonne durchgeführt.

### Beispiel 1

In einem 1-dm³-Kolben mit Rührer, Thermometer und einem Intensivkühler, dessen oberes Ende mit einer auf -20 °c gekühlten Falle verbunden ist, werden unter Stickstoff 500 cm³ wasserfreies N,N-Dimethylacetamid, 480,6 g (1,31 mol) sorgfältig getrocknetes Kalium-perfluor-2-methyl-3-oxa-hexanoat (CF₃CF₂CF₂-O-CF(CF₃)-Co₂K) und 332,5 g Iod (1,31 mol I₂) eingebracht. Die Mischung wird unter Rühren langsam aufgeheizt. Bei 55 °C setzt eine langsame CO₂-Entwicklung ein, die bei 65 °C rasch wird.

Man läßt 90 Min. bei dieser Temperatur reagieren und heizt dann bis 100 °C auf. Nach einstündigem Rühren bei 100 °C ist die Gasentwicklung beendet. Nach Abkühlen der Mischung auf Raumtemperatur wird der Inhalt von Kolben und Kältefalle in 2 l Wasser eingerührt. Die sich abscheidende Unterphase wird abgetrennt und dreimal mit je 800 cm³ Wasser ausgeschüttelt. Nach Trocknen über einem Molekularsieb mit Porengröße 0,4 nm erhält man 495 g Rohprodukt, welches bei Normaldruck destilliert wird. Nach einem Vorlauf, der hauptsächlich aus Perfluor-2-hydro-3-oxa-hexan besteht, erhält man reines, flüssiges Perfluor-2-iod-3-oxa-hexan (Kp. 80 bis 82 °C), das sich am Licht rosa färbt. Ausbeute: 472,2 g (87,5 %).

| Elementaranalyse: | | | |
|---|---|---|---|
| Berechnet: | C 14,58 %, | F 50,74 %; | I 30,80 % |
| Gefunden: | C 14,7 %, | F 50,6 %, | I 30,5 % |

¹³C-NMR:

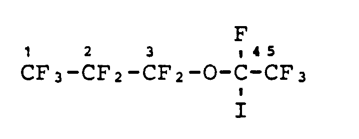

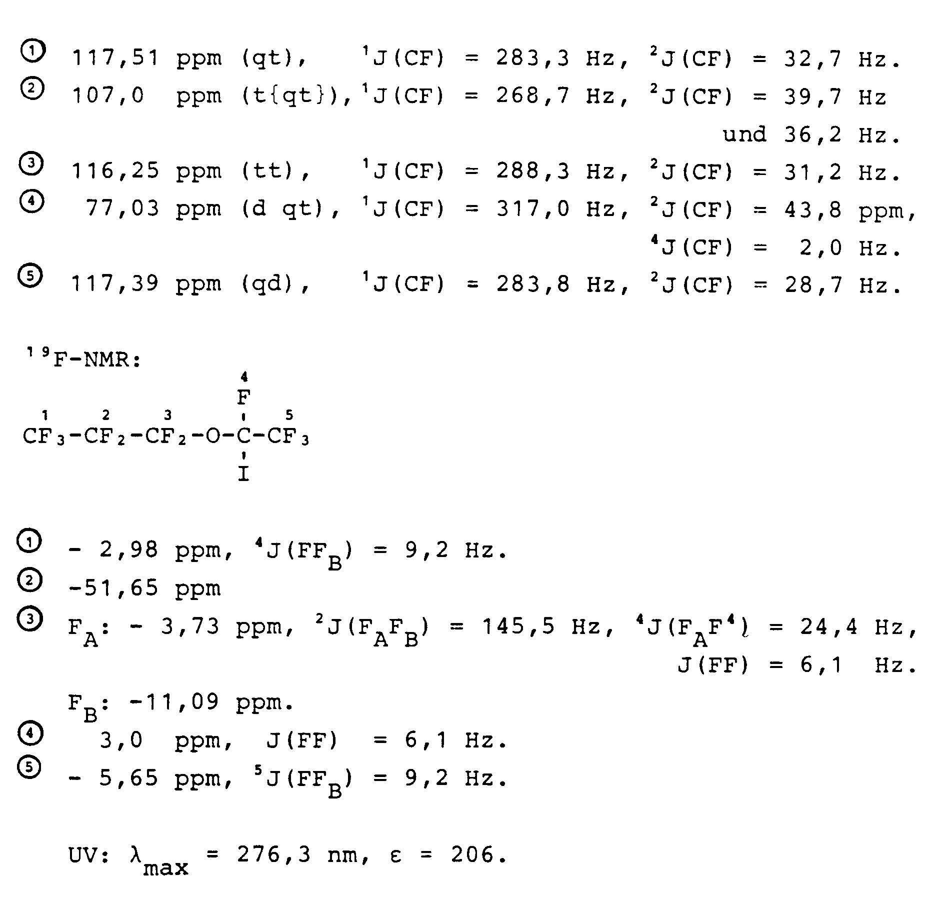

### Beispiel 2:

Eine Mischung aus 368,2 g (1,0 mol) trockenem CF₃CF₂CF₂-O-CF(CF₃)-CO₂K, 256,4 g (1,01 mol) Iod und 500 cm³ wasserfreiem Tetramethylensulfon wird analog Beispiel 1 umgesetzt. Bei 75 bis 80 °C läuft unter gleichmäßiger CO₂-Entwicklung die leicht exotherme Reaktion ab. Nach 30 Min. erhöht man die Temperatur auf 100 °C und läßt noch 1 h nachreagieren. Die Aufarbeitung erfolgt wie im Beispiel 1 beschrieben und liefert 361 g Rohprodukt, aus dem durch destillative Reinigung 338 g reines Perfluor-2-iod-3-oxa-hexan (82,0 % Ausbeute) erhalten werden.

### Beispiel 3

8,9 g (0,02 mol) sorgfältig getrockentes Methyltriethylammonium-perfluor-2-methyl-3-oxa-hexanoat und 5,1 g (0,02 mol) gepulvertes Iod werden unter trockenem Argon sorgfältig vermischt und in einen 50-cm³-Rundkolben gegeben. Man erhitzt die Mischung eine Stunde auf 120 bis 130 °C und leitet das entstehende Abgas durch zwei Kühlfallen (0 °C und -20 °C). Die vereinigen Kondensate werden mit Wasser ausgeschüttelt, die organische Phase wird getrocknet und über eine Mikro-Spaltrohrkolonne fraktioniert. Man erhält 4,3 g (52,4 %) Perfluor-2-iod-3-oxa-hexan.

### Beispiel 4

In der in Beispiel 1 beschriebenen Apparatur werden zu einer Mischung aus 368,2 g (1,0 mol) trockenem CF₃CF₂CF₂-O-CF(CF₃)-CO₂K und 350 cm³ wasserfreiem N,N-Dimethylacetamid 159,8 g (1,0 mol) Brom innerhalb von 30 Min. unter Rühren zugetropft. Man rührt anschließend die Mischung 2,5 h bei 60 °C, wonach die Gasentwicklung beendet ist, kühlt auf Raumtemperatur ab und schüttelt dreimal mit je 1 l Wasser aus. Das mit Molekularsieb (Porenweite 0,4 nm) getrocknete Rohprodukt (315 g) wird bei Normaldruck fraktioniert. Hauptlauf: 285 g Perfluor-2-brom-3-oxa-hexan (Kp. 63 bis 64 °C, Reinheit nach GC: 99,9 %, 78,1 % Ausbeute).

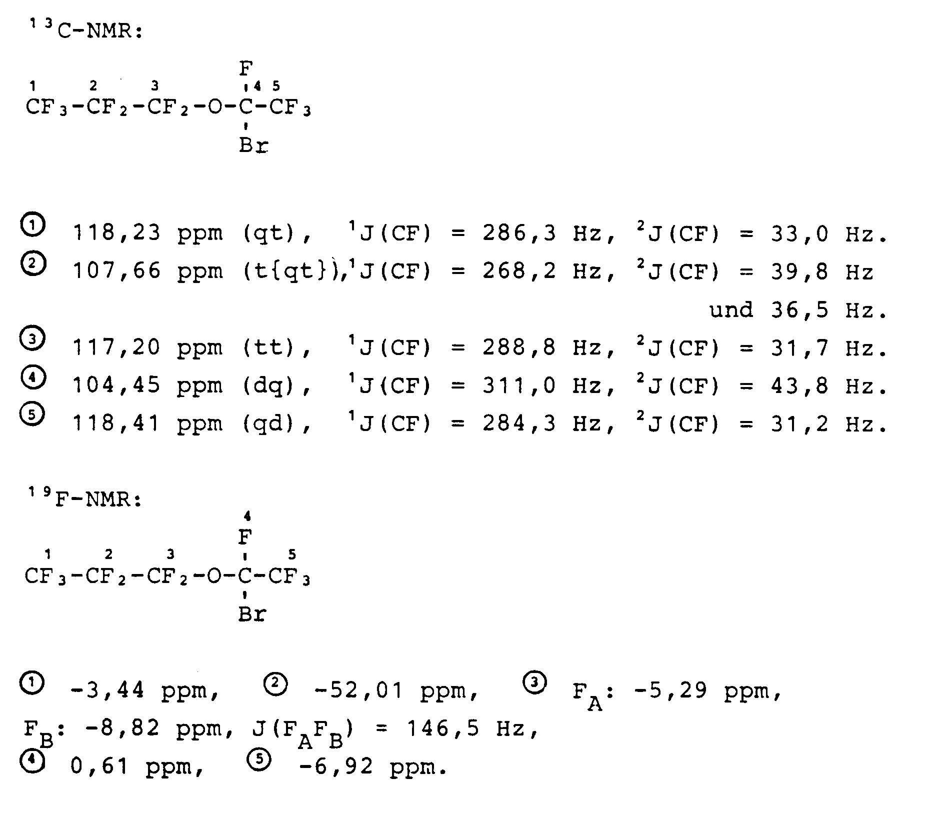

### Beispiel 5

Ein 250 cm³ Autoklav aus Edelstahl wird mit 55,2 g (0,15 mol) CF₃CF₂CF₂OCF(CF₃)-CO₂K, 80 cm³ 1-Chlorperfluoroctan und 13,5 g (0,19 mol) Chlor befüllt und zunächst 2 h bei 120 °C, dann 2 h bei 140 °C geschüttelt. Nach Abkühlen auf Raumtemperatur wird das Reaktionsgemisch dreimal mit je 150 cm³ eiskaltem Wasser ausgeschüttelt und anschließend bei Normaldruck destilliert. Man erhält 26,6 g Perfluor-2-chlor-3-oxahexan (farblose Flüssigkeit, Kp. 56 °C). Ausbeute 55,3 %.

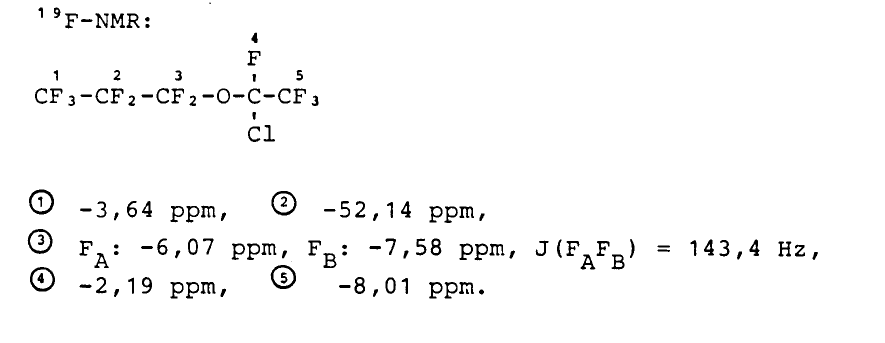

### Beispiel 6

Eine Mischung aus 106,8 g (0,2 mol) trockenem Kaliumperfluor-2,5-dimethyl-3,6-dioxa-nonanoat und 200 cm³ wasserfreiem N,N-Dimethylacetamid wird unter trockenem Stickstoff mit einem Molekularsieb (Porenweite 0,3 nm) unter gelegentlichem Schütteln währed 12 h behandelt, um restliche Feuchtigkeit zu entfernen. Dann filtriert man über eine Fritte, welche das Molekularsieb zurückhält, aber fein dispergierte Salzanteile durchläßt, und wäscht mit 100 cm³ wasserfreiem N,N-Dimethylacetamid nach. Das Filtrat wird unter Stickstoff in einen Rührapparat mit Intensivkühler, dessen oberes Ende durch ein Ventil verschlossen ist, gegeben und mit 50,76 g (0,2 mol) Iod versetzt. Nun heizt man unter Rühren innerhalb einer Stunde allmählich auf 150 °C auf und läßtbei dieser Temperatur noch 30 Min. nachreagieren.Nach Abkühlen auf Raumtemperatur wird mehrfach mit Wasser ausgeschüttelt und das erhaltene Rohprodukt bei Normaldruck destilliert. Man erhält 84,8 g (73,4 %) der reinen Perfluor-2-iod-5-methyl-3,6-dioxa-nonan (Kp. 145 °C). Das ¹⁹F-NMR-Spektrum bestätigt die Struktur 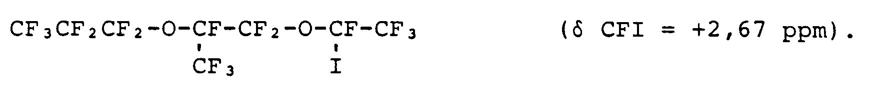
UV: λₘₐₓ = 275,9 nm, ε = 248

### Beispiel 7

Eine Mischung aus 106,8 g (0,2 mol) Kalium-perfluor-2,5-dimethyl-3,6-dioxa-nonanoat (gemahlen und Vakuum-getrocknet), 125 cm³ wasserfreiem Tetramethylensulfon und 33,6 g (0,42 mol) Brom wird unter trockenem Stickstoff innerhalb von 30 Min. auf 85 bis 90 °C erhitzt und 1 h bei dieser Temperatur gerührt. Anschließend rührt man noch 1 h bei 120 bis 125 °C. Das nach Abkühlen und Auswaschen mit Wasser erhaltene Rohprodukt (97,6 g) wird bei Normaldruck fraktioniert. Man erhält 92,1 g (86,7 %) Perfluor-2-brom-5-methyl-3,6-dioxa-nonan (Kp. 125 °C) 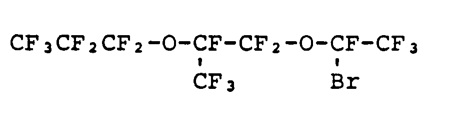
¹⁹F-NMR: δCFBr = -0,45 ppm.

### Beispiel 8

Wie in Beispiel 6 beschrieben wird aus 363,8 g (0,52 mol) Kalium-perfluor-2,5,8-trimethyl-3,6,9-trioxa-dodecanoat und insgesamt 400 cm³ N,N-Dimethylacetamid eine wasserfreie Lösung bereitet. Diese wird unter Ausschluß von Feuchtigkeit mit 83,1 g (0,52 mol) Brom umgesetzt (2 h bei 85 °C). Die Aufarbeitung erfolgt,wie in Beispiel 1 beschrieben,durch Auswaschen und Destillation. Man erhält 270,4 g (74,7 %) einer farblosen Flüssigkeit (Kp. 150 °C/101,3 kPa, beziehungsweise 62 °C/1,1 kPa), deren ¹⁹F-NMR-Spektrum der Struktur 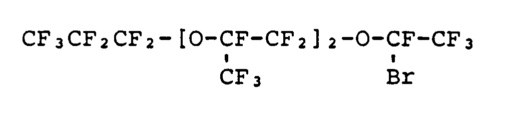
entspricht.

### Beispiel 9

Eine Mischung aus 172,6 g (0,26 mol) Perfluor-2,5,8-trimethyl-3,6,9-trioxa-dodecansäurefluorid und 27,0 g (0,27 mol) Calciumcarbonat-Pulver wird unter Rühren tropfenweise mit 4,68 g (0,26 mol) Wasser versetzt und innerhalb von 1 h auf 100 °C erwärmt. Man trocknet die entstandene Mischung aus Calcium-bis-(perfluor-2,5,8-trimethyl-3,6,9-trioxa-dodecanoat) und Calciumfluorid 10 h bei 100 °C und 10⁻³ kPa. Nach Abkühlen auf Zimmertemperatur verrührt man die Salze unter Argon mit 200 cm³ wasserfreiem Dimethylacetamid und tropft anschließend bei 50 bis 60 °C 41,6 g Brom (0,26 mol Br₂) zu. Dann rührt man noch 1,5 h bei 80 °C. Nach Abkühlen auf 20 °C wird die Mischung mit Wasser verdünnt, die Unterphase wird noch zweimal gewaschen und dann filtriert. Durch fraktionierte Destillation erhält man 129,6 g Perfluor-2-brom-5,8-dimethyl-3,6,9-trioxa-dodecan (71,5 % Ausbeute), dessen ¹⁹F-NMR-Spektrum identisch mit dem Spektrum des in Beispiel 8 erhaltenen Produkts ist.

### Beispiel 10

Wie in Beispiel 6 beschrieben, wird eine Lösung von 107,1 g (0,22 mol) Kalium-perfluor-2-(3′,6′-dimethyl-2′-oxi-1,4-dioxan)-propionat in insgesamt 400 cm³ wasserfreiem N,N-Dimethylacetamid hergestellt. Diese wird unter trockenem Stickstoff gerührt und mit 50,8 g (0,22 mol) Iod versetzt. Man läßt zunächst 1 h bei 30 bis 40 °C, dann noch 30 Min. bei 80 °C reagieren.

Nach Abkühlen auf Raumtemperatur und Isolierung des Rohproduktes durch mehrfaches Ausschütteln mit Wasser wird bei reduziertem Druck fraktioniert.
69,3 g Hauptlauf: Kp. 51 bis 52 °C/13,3 kPa,
Iod-Analyse: Berechnet: 22,8 %,
Gefunden: 17,5 %
¹⁹F-NMR: δCFI = 3,15 ppm.
UV: λₘₐₓ = 276,8 nm.

Aus dem ¹⁹F-NMR-Spektrum ist ersichtlich, daß das Produkt 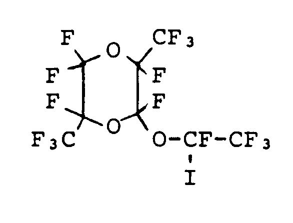
mit Perfluor-2-(1′-hydro-ethoxy)-3,6-dimethyl-1,4-dioxan verunreinigt ist.

## Patentansprüche

1. Verfahren zur Herstellung einer Verbindung der Formel 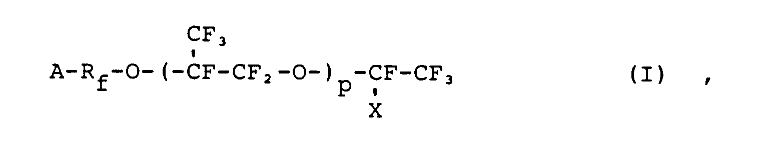 in der bedeuten:
A = H; F; Cl; Br; I oder 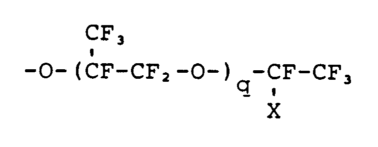
R_{f} einen Perfluoralkylenrest mit 2 bis 10 Kohlenstoffatomen oder A und R_{f} zusammen einen Perfluor-3,6-dimethyl-1,4-dioxanyl-(2)-Rest,
p = Null oder eine Zahl von 1 bis 10,
q = Null oder eine Zahl von 1 bis 10 und
X = Cl; Br oder I
durch Umsetzung eines Salzes einer Verbindung der Formel 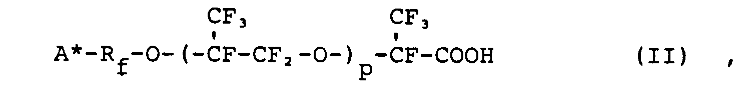 in der bedeuten:
A*= H; F; Cl; Br; I oder 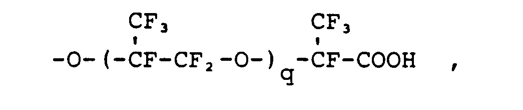
R_{f} denselben Perfluoralkylenrest wie oben oder A* und R_{f} zusammen einen Perfluor-3,6-dimethyl-1,4-dioxanyl-(2)-Rest,
p und q haben die gleiche Bedeutung wie oben
mit einem Halogen bei Zimmertemperatur oder erhöhter Temperatur in Abwesenheit von Wasser, dadurch gekennzeichnet, daß als Salz ein Alkali-, Erdalkali-oder quaternäres Ammoniumsalz der Verbindung der Formel (II) und als Halogen Chlor, Brom, Iod oder mindestens eine Verbindung, die unter den gewählten Reaktionsbedingungen Chlor, Brom oder Iod entwickelt, eingesetzt werden.

2. Verfahren nach Anspruch 1, dadurch gekennzeichnet, daß als Salz der Verbindung der Formel (II) eine Verbindung folgender Formel eingesetzt wird: 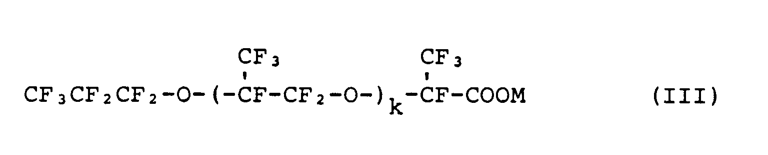 in der bedeuten:
k = Null oder eine Zahl von 1 bis 6,
M = Li; Na; K; 1/2 Ca; 1/2 Mg oder quaternäres Ammonium

3. Verfahren nach Anspruch 1 oder 2, dadurch gekennzeichnet, daß je mol des Salzes der Verbindung der Formel (II) 1 bis 1,2 mol Cl₂; Br₂ oder I₂ verwendet werden.

4. Verfahren nach einem oder mehreren der Ansprüche 1 bis 3, dadurch gekennzeichnet, daß die Umsetzung in mindestens einem polaren, aprotischen Lösungsmittel, das unter den Reaktionsbedingungen flüssig ist, durchgeführt wird.

5. Verfahren nach einem oder mehreren der Ansprüche 1 bis 3, dadurch gekennzeichnet, daß die Umsetzung in mindestens einer weitgehend fluorierten Verbindung als Lösungsmittel durchgeführt wird, die unter den Reaktionsbedingungen flüssig ist und an der Reaktion nicht teilnimmt.

6. Verfahren nach Ansprüchen 4 oder 5, dadurch gekennzeichnet, daß je mol des Salzes der Verbindung der Formel (II) 100 bis 2 000 g des Lösungsmittels verwendet werden.

7. Verfahren nach einem oder mehreren der Ansprüche 1 bis 6, dadurch gekennzeichnet, daß die Umsetzung des Salzes der Verbindung der Formel (II) bei einer Temperatur von 20 bis 150 °C durchgeführt wird.

8. Verfahren nach einem oder mehreren der Ansprüche 1 bis 7, dadurch gekennzeichnet, daß das Kaliumsalz der Verbindung der Formel (II) eingesetzt wird.

## Claims

1. A process for preparing a compound of the formula 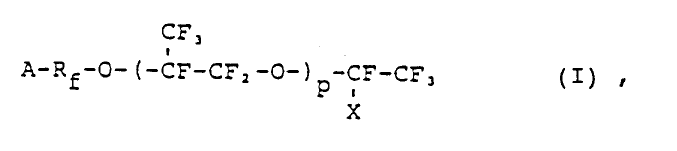 in which:
A is H; F; Cl; Br; I or 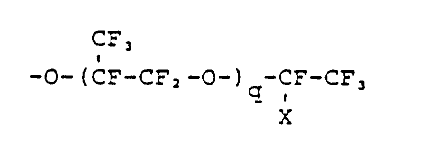
R_{f} is a perfluoroalkylene radical having 2 to 10 carbon atoms, or A and R_{f} together form a perfluoro-3,6-dimethyl-1,4-dioxan-2-yl radical,
p is zero or a number from 1 to 10,
q is zero or a number from 1 to 10 and
X is Cl; Br or I
by reacting a salt of a compound of the formula 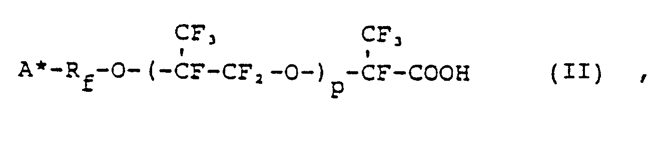 in which
A* is H; F; Cl; Br; I or 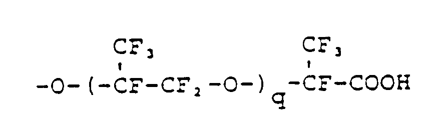
R_{f} is the same perfluoroalkylene radical as above, or A* and R_{f} together form a perfluoro-3,6-dimethyl-1,4-dioxan-2-yl radical, and
p and q are as defined above,
with a halogen at room temperature or elevated temperature in the absence of water, wherein the salt used is an alkali metal, alkaline earth metal or quaternary ammonium salt of the compound of the formula (II) and the halogen used is chlorine, bromine, iodine or at least one compound that evolves chlorine, bromine or iodine under the chosen reaction conditions.

2. The process as claimed in claim 1, wherein as salt of the compound of the formula (II), a compound of the following formula is used: 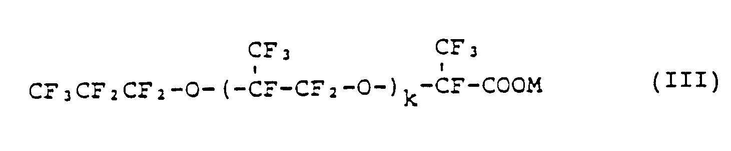 in which:
k is zero or a number from 1 to 6,
M is Li; Na; K; 1/2 Ca; 1/2 Mg or quaternary ammonium.

3. The process as claimed in claim 1 or 2, wherein 1 to 1.2 mol of Cl₂; Br₂ or I₂ are used per mole of the salt of the compound of the formula (II).

4. The process as claimed in one or more of claims 1 to 3, wherein the reaction is carried out in at least one polar, aprotic solvent that is liquid under the reaction conditions.

5. The process as claimed in one or more of claims 1 to 3, wherein the reaction is carried out in, as solvent, at least one substantially fluorinated compound that is liquid under the reaction conditions and does not participate in the reaction.

6. The process as claimed in claim 4 or 5, wherein 100 to 2000 g of the solvent is used per mole of the salt of the compound of the formula (II).

7. The process as claimed in one or more of claims 1 to 6, wherein the reaction of the salt of the compound of the formula (II) is carried out at a temperature of from 20 to 150°C.

8. The process as claimed in one or more of claims 1 to 7, wherein the potassium salt of the compound of the formula (II) is used.

## Revendications

1. Procédé de préparation de composés de formule 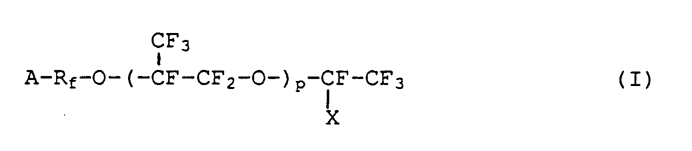 (dans laquelle :
A représente H; F; Cl; Br; I ou un groupe 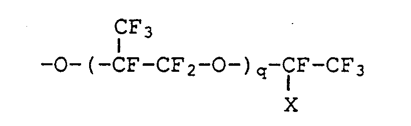
R_{f} est un radical perfluoralkylène ayant de 2 à 10 atomes de carbone ou bien A et R_{f} forment ensemble un radical perfluoro-3,6-diméthyl-1,4-dioxanyle-(2),
p est un nombre de 0 à 10,
q est également un nombre de 0 à 10 et
X désigne Cl; Br ou I)
par réaction d'un sel d'un composé de formule : 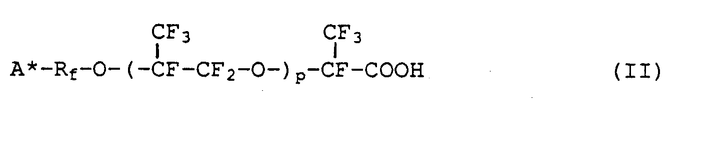 (dans laquelle :
A* représente H; F; Cl; Br; I ou un groupe 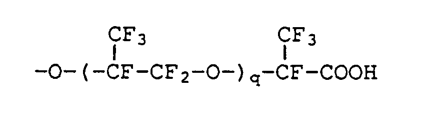
R_{f} est le même radical perfluoralkylène que ci-dessus ou bien A* et R_{f} forment ensemble un radical perfluoro-3,6-diméthyl-1,4-dioxanyle-(2), et
p et q sont les mêmes nombres que ci-dessus)
avec un halogène à la température ambiante ou à une température plus élevée et en milieu anhydre, procédé caractérisé en ce que le sel du composé de formule II que l'on fait réagir est un sel de métal alcalin ou alcalinoterreux ou un sel d'ammonium quaternaire et l'halogène est le chlore, le brome ou l'iode ou bien on utilise à la place un ou plusieurs composés libérant du chlore, du brome ou de l'iode dans les conditions réactionnelles appliquées.

2. Procédé selon la revendication 1, caractérisé en ce que le sel du composé de formule II est un composé de formule : 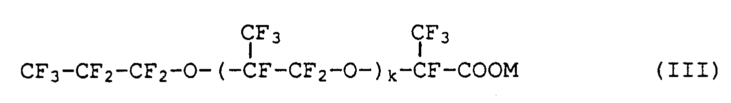 k étant un nombre de 0 à 6 et
M désignant Li; Na; K; 1/2 Ca; 1/2 Mg ou un ammonium quaternaire.

3. Procédé selon la revendication 1 ou 2, caractérisé en ce que l'on met en réaction de 1 à 1,2 mol de Cl₂; Br₂ ou I₂ par mol du sel du composé de formule II.

4. Procédé selon une ou plusieurs des revendications 1 à 3, caractérisé en ce que l'on effectue la réaction dans un ou plusieurs solvants polaires aprotiques, qui sont à l'état liquide dans les conditions réactionnelles.

5. Procédé selon une ou plusieurs des revendications 1 à 3, caractérisé en ce que l'on effectue la réaction dans un un ou plusieurs composés fortement fluorés comme solvants, qui sont à l'état liquide dans les conditions réactionnelles et qui ne participent pas à la réaction.

6. Procédé selon la revendication 4 ou 5, caractérisé en ce que l'on opère avec de 100 à 2 000 g du solvant par mol du sel du composé de formule II.

7. Procédé selon une ou plusieurs des revendications 1 à 6, caractérisé en ce que l'on fait réagir le sel du composé de formule Il à une température de 20 à 150 °C.

8. Procédé selon une ou plusieurs des revendications 1 à 7, caractérisé en ce que l'on utilise le sel de potassium du composé de formule II.
